# DEMANDE DE BREVET EUROPEEN

(11) **EP 2 591 815 A1**
(43) Date de publication de la demande: **15.05.2013**
(21) Numéro de dépôt: 11188705.5
(22) Date de dépôt: 10.11.2011
(51) Int. Cl.: A61M 5/158, A61M 5/142

(54) **Ensemble patch & set d'infusion**

(71) Demandeur: Debiotech S.A., 1004 Lausanne (CH)
(72) Inventeur: Magnenat, Olivier, 1004 Lausanne (CH); Neftel, Frédéric, 1005 Lausanne (CH)
(74) Mandataire: Grosfillier, Philippe

(57) **Abrégé**

Ensemble comprenant un set d'infusion (1) et un patch (2) destinés à accueillir une pompe (9) pour l'administration transcutanée d'un produit, ledit patch (2) comprenant une partie adhésive (10) et une partie non-adhésive (11), l'ensemble étant caractérisé par le fait que ledit set comprend des éléments de guidage de patch (8) et des éléments coulissants (6) destinés à glisser le long des éléments de guidage (8).

## Description

### Domaine de l'invention

L'invention concerne l'administration transcutanée d'un produit au moyen d'une pompe fixée sur un patch et connectée à un set d'infusion.

Elle concerne plus particulièrement un ensemble constitué d'un patch et d'un set d'infusion.

### Etat de la technique

Des ensembles patch & set d'infusion sont divulgués notamment dans les documents brevet EP 1 970 091 A1 et US 2004/0158207.

L'utilisation de tels ensembles permet de connecter, déconnecter et reconnecter aisément une pompe à proximité du set d'infusion, en s'affranchissant de la sorte d'une tubulure entre la pompe et le set.

La mise en place du patch d'adaptation sur le set d'infusion doit être la plus simple possible pour le patient, mais doit permettre de garantir en tous les cas un positionnement correct.

En règle générale, le patch adhésif est d'abord placé sur la peau du patient, puis une canule ou un set d'infusion est insérée dans le patch qui fait office de connexion fluidique entre la pompe et le patient. Ceci nécessite un inserteur pour la canule, inserteur qui doit s'adapter au patch afin de précisément placer la canule avant son insertion.

### Description générale de l'invention

Les problèmes relevés dans le chapitre précédent peuvent être résolus par la présente invention dont l'objet est décrit dans les revendications.

La présente invention simplifie et améliore le positionnement du patch et/ou du dispositif d'insertion du set d'infusion. Elle offre la possibilité à l'utilisateur d'effecteur initialement une étape d'insertion du set d'infusion sans être gêné par le patch et de placer le set d'infusion dans l'orientation souhaitée. Celui-ci pouvant être mis en place lors d'une deuxième étape. Si un inserteur automatique est utilisé, des éléments de connexion au patch ne sont plus nécessaires : Dans un tel cas, un inserteur simplifié, donc plus économique, peut être utilisé. La présente invention permet en outre de positionner le plus précisément possible le patch après insertion du set d'infusion avant de mettre en contact sa zone adhésive avec la peau.

Le verrouillage du patch et du set d'infusion garantit également pour chaque connexion de la pompe avec le set d'infusion que l'aiguille de la pompe soit alignée avec le septum du set et ce même si le site choisi par le patient n'est pas absolument plat.

Etant donné que l'ensemble selon l'invention garantit une mise en place optimale du patch par rapport au set d'infusion, un dispositif de détection de la connexion effective de la pompe d'infusion au set peut être considéré via une détection de connexion de la pompe au patch. A titre d'exemple, la pompe peut détecter (par un détecteur à effet hall) la présence d'un aimant situé sur le patch, indiquant ainsi une connexion correcte au set d'infusion. Du fait du verrouillage du set d'infusion au patch, un aimant placé indifféremment sur le set d'infusion ou sur le patch aura la même fonction, sachant qu'il peut être préférable de placer le dit aimant sur le patch.

Le set d'infusion selon l'invention peut ainsi être mis en place par le patient, avec ou sans inserteur, puis, dans un second temps, le patch est positionné. Une fois ces deux éléments placés, la pompe peut être aisément connectée ou déconnectée de l'ensemble en fonction des activités du patient.

La présente invention permet notamment de faciliter la mise en place d'un patch et de garantir que celui-ci soit correctement en position.

Elle sera mieux comprise ci-après au moyen d'un mode de réalisation non-limitatif illustré par quelques figures.

### Description détaillée de l'invention

La figure 1 représente un set d'infusion (sans canule) selon l'invention.
La figure 2 représente un patch selon l'invention pouvant être utilisé avec le set de la figure 1.
La figure 3 illustre l'approche du patch de la figure 2 vers le set de la figure 1.
La figure 4 montre la zone de contact des deux pièces et la direction du glissement du patch.
La figure 5 présente la forme évasée d'une zone du patch dans laquelle sont disposées deux ergots.
La figure 6 illustre un mécanisme permettant de prévenir un rabattement accidentel du patch contre la peau.
La figure 7 montre le positionnement du patch qui précède sa rotation vers sa position définitive.
La figure 8 présente le patch dans sa position définitive.
La figure 9 présente la zone de contact entre le set et le patch.
La figure 10 représente l'ensemble selon l'invention sur lequel est fixée une pompe.
La figure 11 illustre une coupe d'une partie de l'objet de la figure 10.

### Références numériques utilisées dans les figures :

- 1.: Set d'infusion
- 2.: Patch
- 3.: Face supérieure du patch
- 4.: Face inférieure du patch
- 5.: Septum
- 6.: Ergots
- 7.: Espace inter-ergots
- 8.: Rails
- 9.: Pompe
- 10.: Partie adhésive
- 11.: Partie non-adhésive
- 12.: Butée 13. Rampe 14. Zone de saisie
- 15.: Aimant
- 16.: Capteur 17. Passage
- 18.: Décrochement

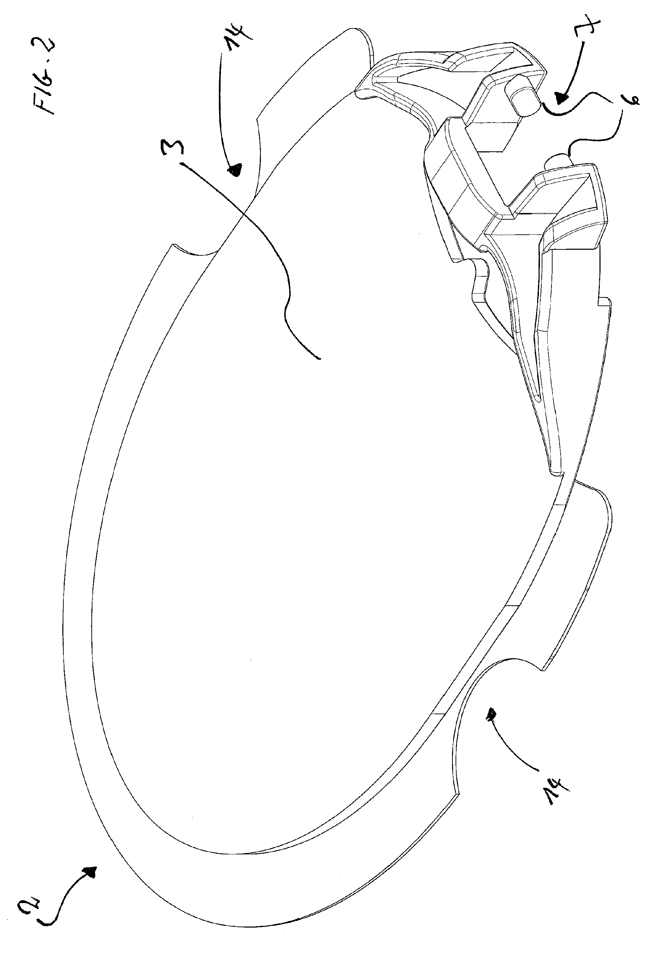

Afin de faciliter la compréhension de l'invention, le patch **2** présenté notamment sur la figure 2 ne comprend pas des moyens pour assurer la fixation d'une pompe **9**.

A noter également l'absence d'illustration de la zone adhésive sur la face inférieure 4 du patch **2**. Avant usage cette zone adhésive est recouverte d'un film protecteur.

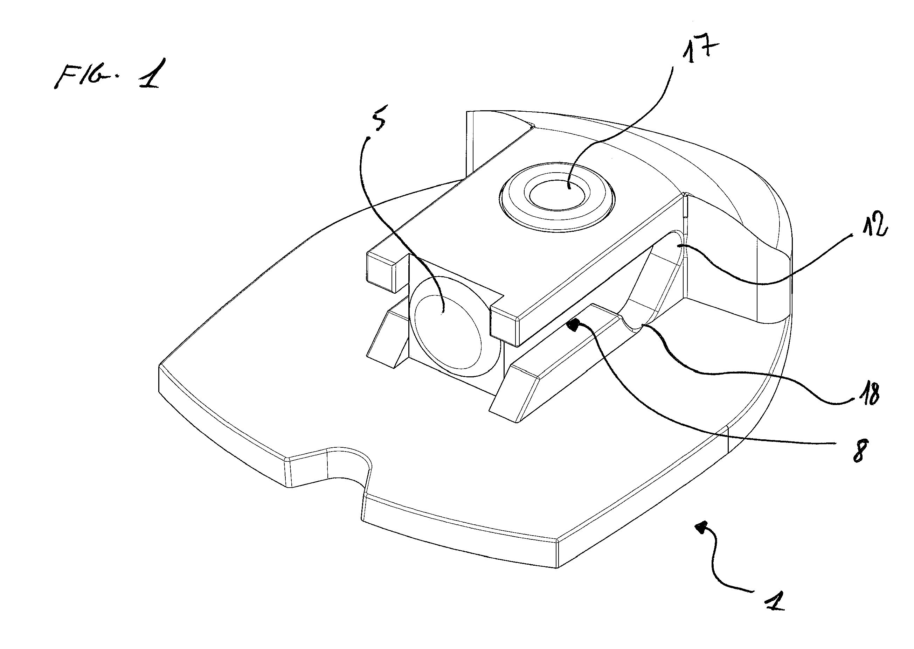

Le set d'infusion **1** illustré notamment sur la figure 1 comprend un septum **5** qui obture un conduit (non-illustré) assurant une communication fluidique entre une pompe et une canule. Le set d'infusion **1** comprend également un passage **17** pour l'introduction et le retrait d'un mandrin. Il comprend enfin des éléments de guidage de patch qui se présentent sous la forme rails **8**.

La figure **2** présente un patch **2** compatible avec le set d'infusion **1** de la figure 1. La face supérieure **3** du patch **2** est destinée à recevoir une pompe. Le patch **2** comporte deux zones de retrait **14** pour faciliter la saisie du patch **2** avec les doigts. La partie avant du patch **2** comporte une zone évasée **7** au bord de laquelle sont disposés deux ergots **6** destinés à coulisser dans les éléments de guidage **8** du set d'infusion **1**.

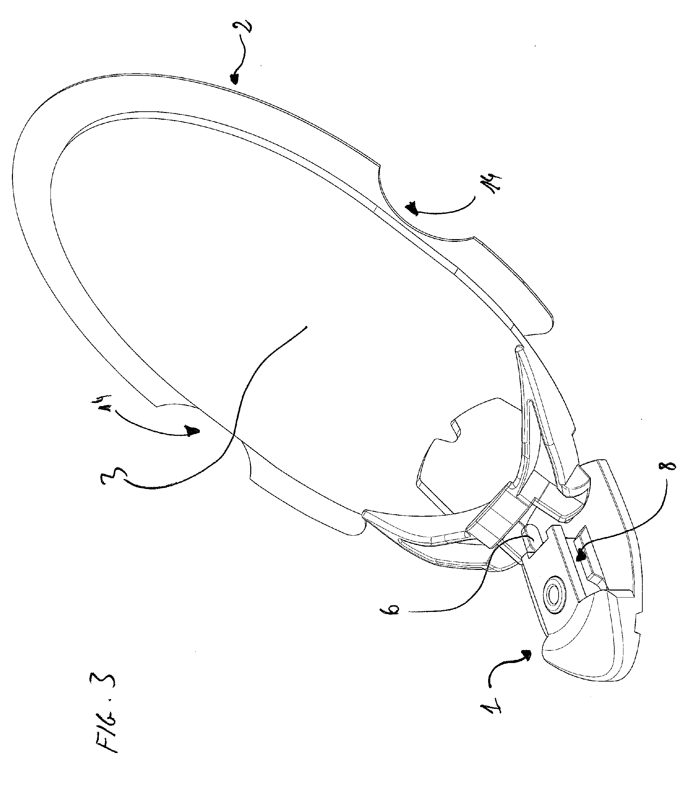

Suite au retrait de son film protecteur disposé sur sa face inférieure **4** (voir figure 3), le patch **2** est approché du set d'infusion 1 tout en gardant un certain angle avec la surface de la peau. Idéalement un tel angle est de 30° à 60°, mais de préférence supérieur à 15°. L'extrémité du patch **2** vient en appui sur le set d'infusion 1 et offre ainsi une première référence de placement.

Le patch **2** est maintenu en contact avec le set d'infusion **1** et peut être glissé en direction du septum **5**. La forme évasée de la partie avant **7** du patch **2** lui permet de facilement faire passer la partie septum **5** du set d'infusion **1** en son centre.

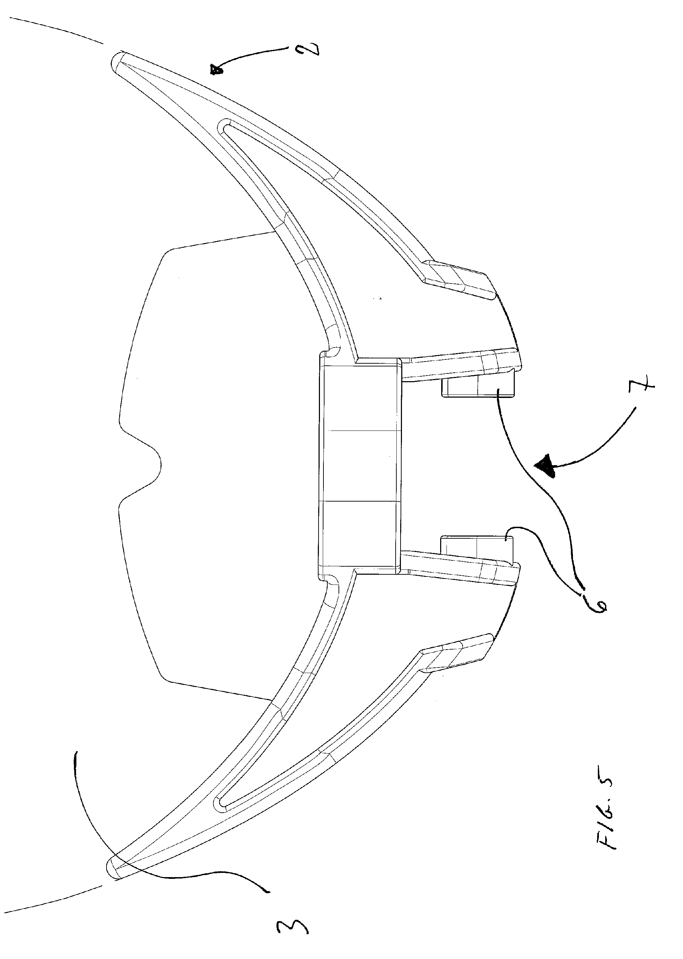

Cette même forme **7** (voir figure 5) permet en outre d'affiner le positionnement du patch **2** à mesure que celui-ci est glissé.

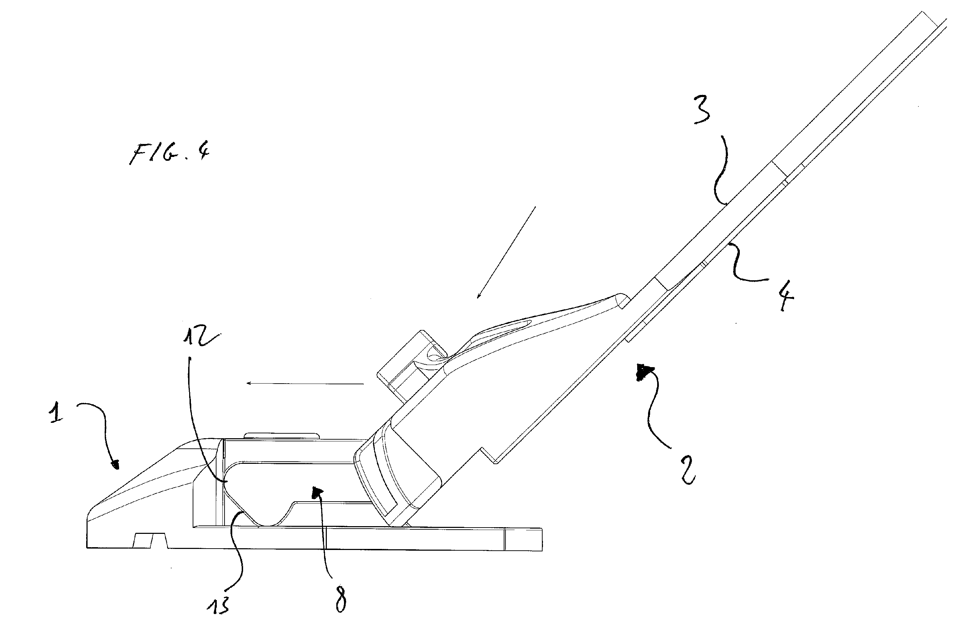

Deux ergots **9** disposés sur le patch **2** (cf. figure 4) vont ensuite glisser entre des rails **8** du set 1, en direction du septum 5.

Que ce soit par un design spécifique des ergots **6** et/ou des rails **8** ou par tout autre système, il est impératif que le patch **2** ne puisse être rabattu contre la peau tant qu'il n'a pas atteint sa position définitive. En effet, un patch **2** mal positionné rendrait impossible la connexion d'une pompe avec le set d'infusion 1.

L'ensemble selon l'invention tel qu'illustré évite ce rabattement accidentel du patch **2** pendant son déplacement vers sa position définitive.

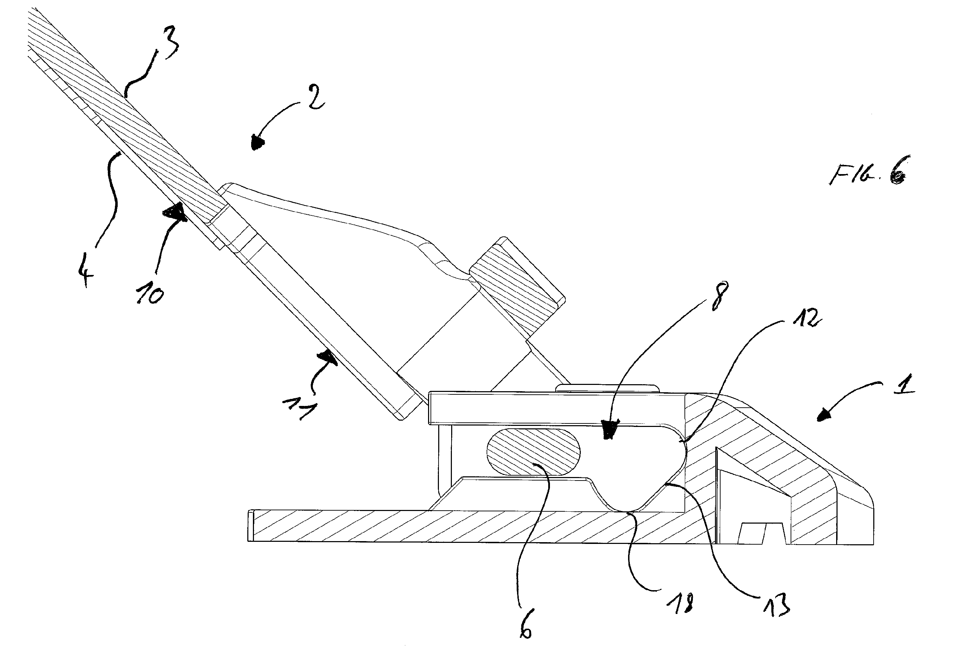

Comme on peut le voir en particulier sur la figure 6, une fois les ergots **6** engagés dans les rails **8**, le patch **2** ne peut que translater dans le sens des rails **8**. Comme les ergots **6** sont orientés selon une direction oblique par rapport à l'orientation principale du patch **2**, ce dernier ne peut se déplacer qu'en restant selon une direction oblique par rapport à la surface de la peau lorsqu'il se déplace dans les rails **8**.

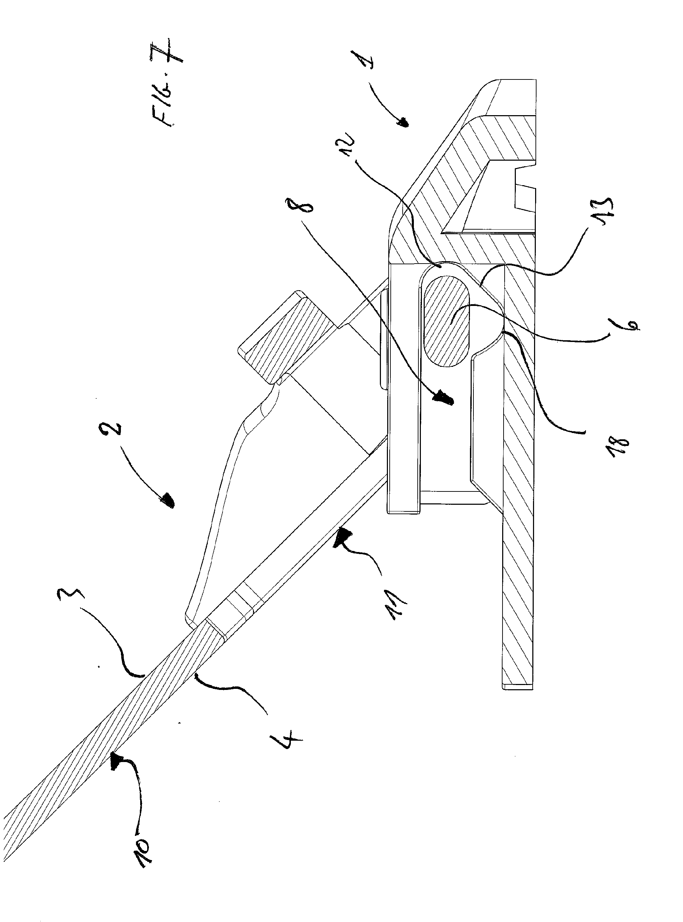

La figure 7 illustre la fin de la course du patch **2**, contre une butée **12**, avant son abaissement. Celui-ci est rendu possible car l'extrémité des rails **8** comporte des décrochements **18** dans lesquels peuvent se loger les ergots **6** selon une direction oblique par rapport à la surface de la peau, l'angle ainsi formé par les ergots **6** est sensiblement équivalent à l'angle de rotation effectué par le patch **2** pour adhérer à la peau.

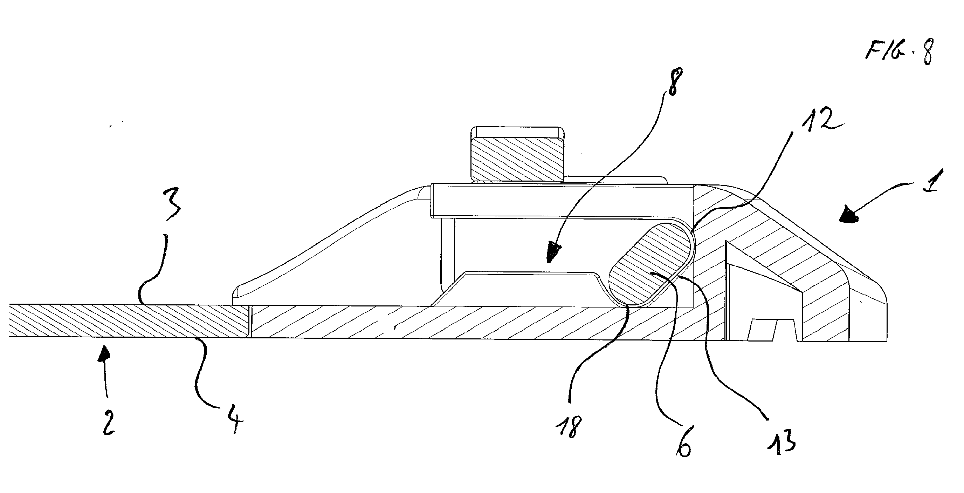

La figure 8 montre le patch 2 dans sa position définitive.

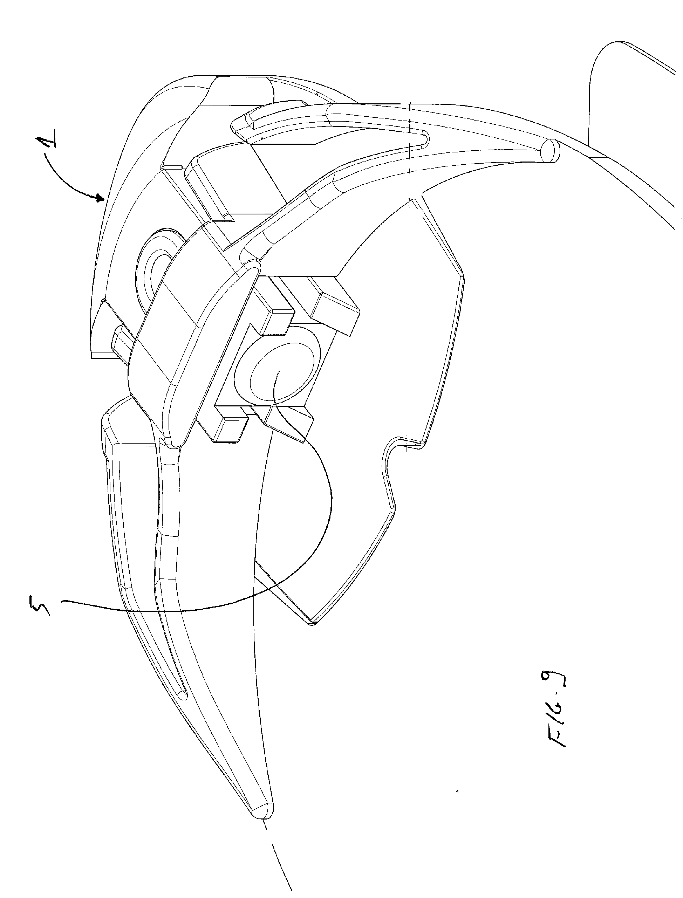

La figure 9 illustre la zone de contact du patch **2** avec le set d'infusion **1**, prête à recevoir une pompe.

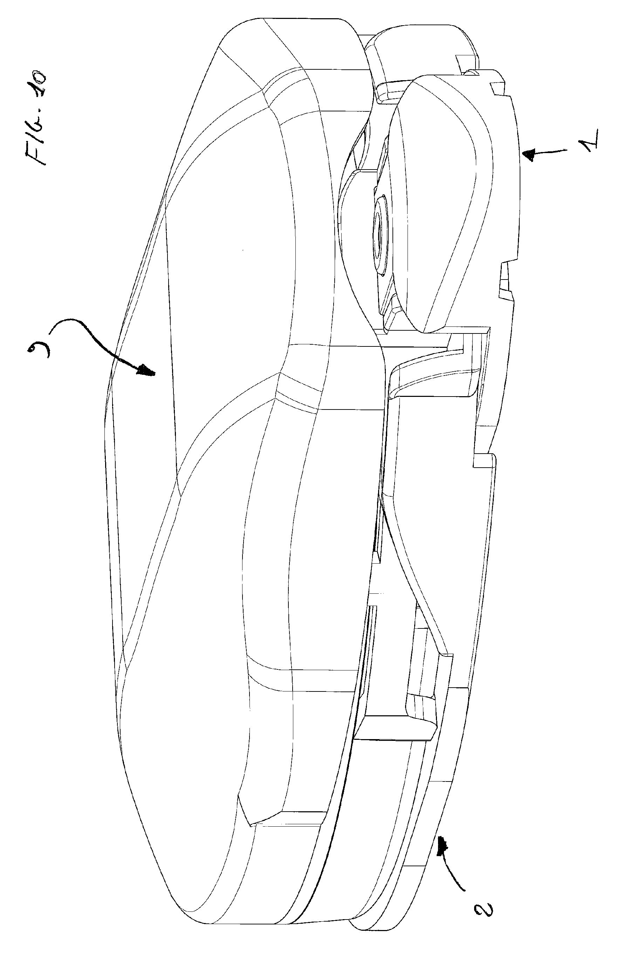

La figure 10 montre une pompe **9** fixée sur le patch **2**.

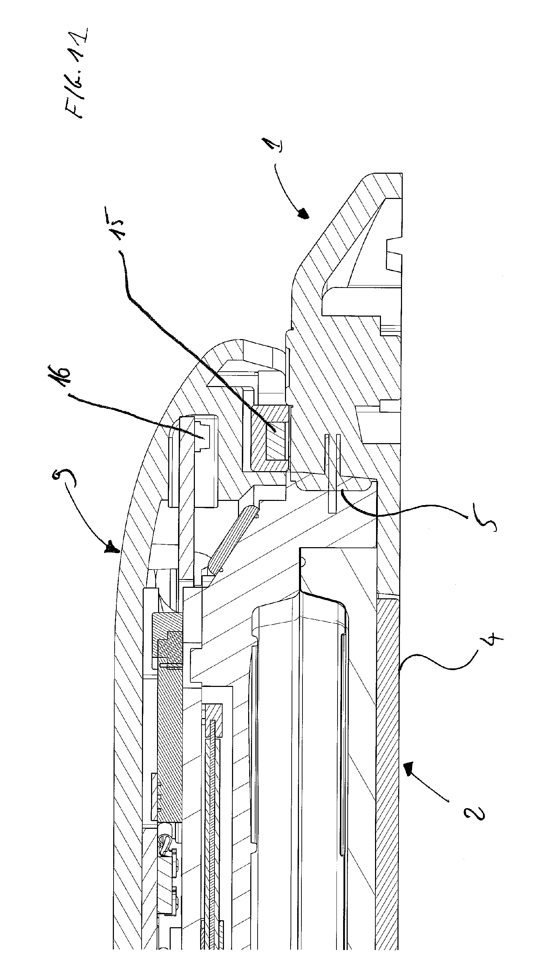

La figure 11 illustre également un aimant **15** disposé sur le set 1 et un détecteur à effet Hall **16** fixé à la pompe, ces deux éléments étant positionnés de manière à vérifier l'état de la connexion entre la pompe **9** et le set d'infusion 1. Toute autre forme de réalisation de ce système de détection de connexion peut être envisagée. L'aimant peut p.ex. être fixé à la pompe **9** et le détecteur au set d'infusion **1**, voire au patch **2**.

L'ensemble selon l'invention permet d'assurer que le patch **2** soit positionné correctement sur le set d'infusion **1**, sans risque de coller le patch **2** sur la peau du patient par inadvertance. Le placement définitif du patch **2** sur la peau ne peut être obtenu que par rotation du patch **2** selon un axe dont la direction est définie par les ergots **6** et uniquement lorsque ces derniers sont logés dans les décrochements **18**. En définitive, le patch **2** est arrimé au set d'infusion **1** avec un angle assurant une position correcte de la pompe **9** venant se connecter ultérieurement au set d'infusion 1 au moyen du patch **2**.

La présente invention offre notamment les avantages suivants :
- Le guidage ergots/rails permet de rendre définitivement solidaire le patch et le set d'infusion même si le patch n'a pas d'adhésif au niveau de la surface proche du set. La courbure du site d'infusion ne joue plus un rôle pour garantir une connexion pompe/set correct.
- Les faux positifs sont exclus. Le patient ne peut pas se trouver dans un cas de figure où il pense avoir bien mis son patch alors que ce n'est pas le cas.
- Utilisation d'un sous-ensemble de deux pièces dont la précision de positionnement l'une par rapport à l'autre est suffisante pour envisager de détecter avec la pompe seulement la présence du patch et donc pas obligatoirement la présence du set d'infusion.
- Grande facilité de manipulation.
- Le patch ne peut accidentellement être collé prématurément sur la peau du patient avant d'avoir atteint sa position définitive par rapport au set d'infusion.
- Possibilité d'utiliser un inserteur simplifié, p.ex. sans dispositif particulier de référence par rapport au patch. Seule une indication de l'orientation du set d'infusion peut être souhaitable, afin que le patient sache dans quelle direction sera orienté le patch.

## Revendications

1. Ensemble comprenant un set d'infusion (1) et un patch (2) destinés à accueillir une pompe (9) pour l'administration transcutanée d'un produit, ledit patch (2) comprenant une partie adhésive (10) et une partie non-adhésive (11), l'ensemble étant **caractérisé par le fait qu'**il comprend des éléments de guidage de patch (8) et des éléments coulissants (6) destinés à glisser le long des éléments de guidage (8).

2. Ensemble selon la revendication 1 dans lequel la partie non-adhésive du patch (11) comporte les éléments coulissants (6).

3. Ensemble selon la revendication 1 ou 2 dans lequel ledit set (2) comprend des éléments de butée (12) disposés vers l'une des extrémités des éléments de guidage (8) et adaptés de manière à bloquer le déplacement des éléments coulissants (6).

4. Ensemble selon l'une quelconque des revendications précédentes dans lequel les éléments coulissants (6) sont disposés vers un bord du patch (2).

5. Ensemble selon l'une quelconque des revendications précédentes dans lequel les éléments coulissants (6) sont disposés le long d'un axe autour duquel le patch peut pivoter librement une fois que les éléments coulissants se trouvent dans les éléments de guidage.

6. Ensemble selon l'une des revendications 1 à 4 dans lequel les éléments coulissants (6) sont disposés le long d'un axe autour duquel le patch peut pivoter librement seulement lorsque que les éléments coulissants (6) se trouvent en fin de course.

7. Ensemble selon la revendication précédente dans lequel les éléments coulissants (6) sont orientés selon une direction qui forme un angle par rapport à l'orientation principale du patch (2).

8. Ensemble selon la revendication 7 dans lequel ledit angle est d'au moins 15°.

9. Ensemble selon l'une quelconque des revendications précédentes dans lequel les éléments de guidage sont des rails (8) et dans les éléments coulissants sont des ergots (6).

10. Ensemble selon la revendication précédente dans lequel la section de chacun desdits ergots (6) se présente sous la forme d'un rectangle bordé de deux demi-disques.

11. Ensemble selon la revendication 9 ou 10 comprenant deux ergots (6) disposés de chaque côté d'une portion évasée (7) du patch (2).

12. Ensemble selon l'une des revendications précédentes dans lequel les éléments de guidage (8) comprenant des éléments de blocage (13) destinés à bloquer le patch (2) dans sa position définitive.

13. Ensemble selon l'une quelconque des revendications précédentes dans lequel le bord du patch (2) comprend au moins deux zones de retrait (14) destinée à acceuillir les doigts de l'utilisateur.

14. Utilisation d'un ensemble selon l'une quelconque des revendications précédentes comprenant les étapes suivantes :
- Mise en place du set d'infusion (1),
- Retrait du film adhésif du patch (2),
- Mise en contact des éléments coulissants (6) avec les éléments de guidage (8) et déplacement du patch (2) selon une direction transverse par rapport à la surface de la peau,
- Abaissement et mise en contact du patch (2) avec la peau.
